(19) Europäisches Patentamt
European Patent Office
Office européen des brevets

(11) EP 2 059 805 B1

(12) **EUROPEAN PATENT SPECIFICATION**

(45) Date of publication and mention of the grant of the patent:
**30.05.2012 Bulletin 2012/22**

(21) Application number: **07826023.9**

(22) Date of filing: **14.08.2007**

(51) Int Cl.:
***G01N 33/52*** *(2006.01)* ***C12Q 1/04*** *(2006.01)*

(86) International application number:
**PCT/IB2007/053231**

(87) International publication number:
**WO 2008/026119 (06.03.2008 Gazette 2008/10)**

(54) **MICROBE-SENSITIVE INDICATORS AND USE OF THE SAME**

MIKROBENEMPFINDLICHE INDIKATOREN UND VERWENDUNG DAVON

INDICATEURS A SENSIBILITE MICROBIENNE ET UTILISATION ASSOCIEE

(84) Designated Contracting States:
**DE GB**

(30) Priority: **31.08.2006 US 513702**

(43) Date of publication of application:
**20.05.2009 Bulletin 2009/21**

(73) Proprietor: **KIMBERLY-CLARK WORLDWIDE, INC.**
**Neenah, WI 54956 (US)**

(72) Inventors:
- **MARTIN, Stephanie, Michelle**
**Woodstock, Georgia 30188 (US)**
- **MACDONALD, John, Gavin**
**Decatur, Georgia 30033 (US)**
- **SNOWDEN, Hue, Scott**
**Canton, Georgia 30115 (US)**
- **BRANHAM, Kelly, Dean**
**Woodstock, Georgia 30188 (US)**

(74) Representative: **Zimmermann & Partner**
**Postfach 330 920**
**80069 München (DE)**

(56) References cited:
**EP-A- 0 813 850**
**WO-A-2006/065349**
**WO-A-2007/027899**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### BACKGROUND

Health professionals are exposed to a wide variety of pathogenic organisms on a daily basis, and rely heavily on their personal protective equipment to provide a barrier against harmful bodily fluids. Though it may be easy to observe contamination of a gown or drape by blood, these garments may be exposed to other sources of contamination less visible to the naked eye, such as mucus, spit, sputum, tears, and hand contaminants. Furthermore, a contaminant such as blood may be difficult to detect on an item having a very dark color such as black or brown.

Similarly, the detection of harmful levels of microbes in the food processing business is very important in maintaining the health of workers and customers alike. In the food processing industry, bacteria monitoring is critical. The processing of virtually all foods, from meat packing to cheese production, involves monitoring microbe levels in order to ensure the safety of the food supply. The safety of the food supply may be compromised if protective wear worn by food service industry workers becomes contaminated by bacteria that could subsequently be transferred into the food supply. WO 2006/065349 A discloses an elastomeric article that contains a chromogen that undergoes a detectable change in color in the presence of one or more microbes. The chromogen can be a solvatochromic dye (e.g. Reichardt's dye) that undergoes a color change in the presence of bacteria or other microbes. According to WO 2006/065349 A, such dyes may respond to differences in polarity between microbe components (e.g. cell membrane, cytoplasm, etc.) and the environment outside the cell. Alternatively, other mechanisms may be wholly or partially responsible for the interaction between the dye and the microbe, such as acid-base reactions, redox reactions, and so forth.
WO 2007/027899 A, corresponding to EP 1 919 421 A, which is a document under Art. 54 (3) EPC, describes a graphic and/or message display system. The graphic and/or message display system functions to develop over a period of time a hidden graphic or message on an indicator panel or display area. The hidden graphic or message is revealed when an obscuring graphic reacts with a solvent to change color. The graphic and or message display system may be used as a stand-alone device or may be incorporated as part of various articles or products, for instance, as a positive reinforcement or a reminder to perform a task. Methods for using the graphic and/or message display system are also described.

As such, systems and methods for detecting microbial contamination on various surfaces could ultimately help prevent the further spread of germs and disease by causing steps to be taken to reduce or eliminate the transfer of microbes. Such a system or method in a health care setting could also provide assurance to patients that their healthcare provider is taking the proper steps to reduce the risk of contamination by bacteria or other microbes, just as it would to a patron in a food preparation setting.

Therefore, a need exists for a system and method for detecting microbial contamination on a variety of surfaces. The present invention addresses one or more of the aforementioned needs.

### SUMMARY OF THE INVENTION

The systems, devices, and methods described herein can be adapted to a host of potential products and uses for which there may be a need for detecting an exposure to microbial contamination.

In one embodiment, a method of detecting the microbial contamination of a surface includes the steps of providing an article having a surface, the surface having thereon a signal graphic and a masking graphic overlying and obscuring the signal graphic. The masking graphic includes a microbe-sensitive dye. The method further includes the steps of activating the microbe-sensitive dye by contact with a microbial contaminant to expose the signal graphic and detecting the exposed signal graphic. The method may further include the step of taking an action in response to detection of the exposed signal graphic. In one aspect the responsive action includes one or more steps selected from the group consisting of cleaning the surface, disposing of the surface, and initiating an infection control protocol. In a further aspect, the signal graphic and the masking graphic cover less than the entirety of the surface. Optionally, the signal graphic and the masking graphic are visually distinct from a background color of the surface.

In one aspect of the method, a reaction between the microbe-sensitive dye and the microbial contaminant exposes the signal graphic. The reaction between the microbe-sensitive dye and the microbial contaminant may render the masking graphic substantially transparent. In another aspect, the reaction between the microbe-sensitive dye and the microbial contaminant may cause the signal graphic to change color or become colorless. The reaction between the microbe-sensitive dye and the microbial contaminant may occur over a period of time less than about 5 minutes.

In a further aspect of the above method, the surface of the article may be on a major surface of a nonwoven material. The nonwoven material may be selected from the group consisting of film, spunbond, meltblown, SMS, coform, and airlaid materials.

In an even further aspect, the microbe-sensitive dye may include a zwitterionic chromogen. Optionally, the zwitterionic chromogen may include Reichardt's dye, a merocyanine, or a compound having the formula

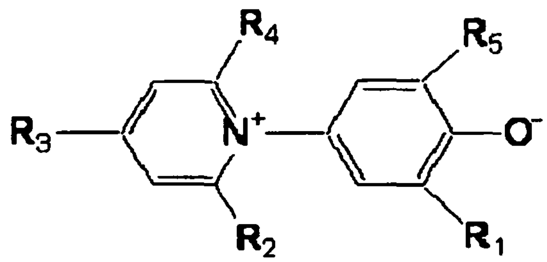

wherein $R_1$, $R_2$, $R_3$, $R_4$, and $R_5$ are selected from the group consisting of phenyl, benzyl, pyridinyl, aryl, heteroaryl, alkyl ($C_1$ - $C_{12}$), cycloalkyl, heterocyclo, halide, chlorine, fluorine, hydrogen, amido, amine, and thiol groups.

In one aspect, the masking graphic and the signal graphic are differently colored.

In another embodiment, a personal care article has a surface, the surface having thereon a signal graphic and a masking graphic overlying and obscuring the signal graphic. The surface is a major surface of a nonwoven material. The nonwoven material is selected from the group consisting of spunbond, meltblown, SMS, coform, and airlaid materials. Desirably, the masking graphic includes a microbe-sensitive dye.

In one aspect, the personal care article may be a glove, a gown, a drape, and so forth.

Additional features and advantages of the microbe contamination detection system and associated articles of manufacture and methods will be disclosed in the following detailed description. It is understood that both the foregoing summary and the following detailed description and examples are merely representative of the invention, and are intended to provide an overview for understanding the invention as claimed.

BRIEF DESCRIPTION OF FIGURES

A full and enabling disclosure of the present invention, including the best mode thereof to one of ordinary skill in the art, is set forth more particularly in the remainder of the specification, including reference to the accompanying figures in which:

FIG. 1 a-c depict a schematic representation of a microbe contamination detection system and use thereof.

Repeat use of reference characters in the present specification and drawings is intended to represent the same or analogous features or elements of the invention.

DETAILED DESCRIPTION OF THE INVENTION

Reference will now be made in detail to various embodiments of the invention, one or more examples of which are set forth below. Each embodiment is provided by way of explanation of the invention, not limitation of the invention. In fact, it will be apparent to those skilled in the art that various modifications and variations may be made in the present invention without departing from the scope or spirit of the invention. For instance, features illustrated or described as part of one embodiment, may be used on another embodiment to yield a still further embodiment. All technical and scientific terms used herein have the usual meaning conventionally understood by persons skilled in the art to which this invention pertains, unless context defines otherwise. The present invention is not necessarily limited to specific compositions, materials, designs or equipment, as such may vary. As used in this specification and the appended claims, the singular forms "a," "an," and "the" include plural referents unless the context clearly dictates otherwise. Thus, it is intended that the present invention cover such modifications and variations as come within the scope of the appended claims and their equivalents.

As used herein, and in the claims, the term "comprising" is inclusive or openended and does not exclude additional unrecited elements, compositional components, or method steps. Accordingly, such term is intended to be synonymous with the words "has", "have", "having", "includes", "including", and any derivatives of these words.

In one embodiment, an article includes a microbial contamination detection system. The microbial contamination detection system includes a surface of the article having thereon a signal graphic and a masking graphic overlying and obscuring the signal graphic. The signal graphic may be, for example, a color, a message, or any other graphic that can be used to convey a signal to the viewer of the graphic. The masking graphic includes a microbe-sensitive dye. The masking graphic at least partially obscures the signal graphic prior to any microbial contamination. Desirably the masking graphic substantially obscures the signal graphic prior to any microbial contamination. Upon contact by a microbial contaminant with the masking graphic, the microbe-sensitive dye changes appearance to expose the underlying signal graphic. Thus, the appearance of the initially obscured signal graphic is activated by a contact with a microbial contaminant, hence resulting in notification to a user or viewer of the article. It is envisioned that the microbial contamination detection system may be used for a variety of applications in which the indication of exposure to a microbial contaminant would be advantageous. For example, the microbial contamination detection system may be used in a health care setting or food preparation setting where the spread of microbial contamination is to be avoided. In other embodiments, associated articles of manufacture and methods for using the microbial contamination detection system to indicate the occurrence of microbial contamination are described.

In one aspect, the masking graphic includes a microbe-sensitive chromogen or dye. The microbe-sensitive dye desirably obscures the signal graphic until the time at which a microbial contamination occurs. Microbe-sensitive dyes are a unique class of dyes that become transparent, become substantially transparent, or undergo a color change when the molecular environment, such as solvent polarity, hydrogen bonding propensity, and so forth, changes. In one embodiment, the microbe-sensitive dye includes a solvatochromic dye. Desirably, the microbe-sensitive dye changes color, becomes substantially transparent, or becomes transparent after exposure to a microbial contaminant.

In another aspect, the microbe-sensitive dye having a first color may be combined with a second dye having a second color to create a dye mixture having a third color. The dye mixture is used to create an initial graphic on a surface of an article. After application of a microbial contaminant to the dye mixture, the color of the graphic changes as the microbe-sensitive dye changes (to create a fourth color) or becomes transparent (to reveal the second color).

Any of a variety of articles, devices, substrates or garments may be incorporated with the microbe contamination detecting system.

The microbe contamination detecting system may be used in a method useful for the visual detection of color changes associated with microbe contamination of the system.

Such a method of detection using the microbe contamination detection system may be useful for use on solid surfaces like, for example, packaging such as facial tissue boxes, stickers, paper, tissues, medical paraphernalia like surgical gloves, surgical gowns and drapes, face masks, head coverings like bouffant caps, surgical caps and hoods, examination and surgical gloves, footwear like shoe coverings, boot covers and slippers, wound dressings, bandages, sterilization wraps, wipers, garments like lab coats, coveralls, aprons and jackets, patient bedding, stretcher and bassinet sheets, food preparation wraps, dish sponges, cloths, door handles, telephones, computer keyboards, computer mice, pens, pencils, notepads, toilet handles, wound dressings, bandages, and toys (e.g. in doctors waiting rooms, daycare facilities).

Substrates onto which the microbe contamination detection system may be applied may therefore include protective and other garments, as well as other articles that may be exposed to microbes like those mentioned below. The microbe contamination detection system may also be incorporated into sponges or dish towels to warn of contamination.

Substrates suitable for use with the microbe contamination detection system include any of those traditionally used for garments and protective apparel including, but not limited to, films, woven and nonwoven fabrics, cellulosic substrates like tissues, paper towels and coform materials, airlaid materials, bonded-carded webs and so forth. Nonexclusive examples of substrates may be found in US patents 4,775,582 and 4,853,281, 4,833,003, and 4,511,488, all assigned to the Kimberly-Clark Corporation.

A nonwoven fabric may be made according to processes like spunbonding, meltblowing, airlaying, bonding and carding, and so forth. Nonwoven fabrics may be made from thermoplastic resins including, but not limited to polyesters, nylons, and polyolefins. Olefins include ethylene, propylene, butylenes, isoprene and so forth, as well as combinations thereof.

"Spunbonded fibers" are small diameter fibers which are formed by extruding molten thermoplastic material as filaments from a plurality of fine, usually circular capillaries of a spinneret with the diameter of the extruded filaments then being rapidly reduced as by, for example, in US Patent 4,340,563 to Appel et al., and US Patent 3,692,618 to Dorschner et al., US Patent 3,802,817 to Matsuki et al., US Patents 3,338,992 and 3,341,394 to Kinney, US Patent 3,502,763 to Hartman, and US Patent 3,542,615 to Dobo et al. Spunbond fibers are generally not tacky when they are deposited onto a collecting surface. Spunbond fibers are generally continuous and have average diameters (from a sample of at least 10) larger than 7 microns, more desirably, between about 10 and 20 microns.

"Meltblown fibers" means fibers formed by extruding a molten thermoplastic material through a plurality of fine, usually circular, die capillaries as molten threads or filaments into converging high velocity, usually hot, gas (e.g. air) streams which attenuate the filaments of molten thermoplastic material to reduce their diameter, which may be to microfiber diameter. Thereafter, the meltblown fibers are carried by the high velocity gas stream and are deposited on a collecting surface to form a web of randomly dispersed meltblown fibers. Such a process is disclosed, for example, in US Patent 3,849,241 to Butin et al. Meltblown fibers are microfibers which may be continuous or discontinuous, are generally smaller than 10 microns in average diameter, and are generally tacky when deposited onto a collecting surface.

As used herein, the term "coform" means a process in which at least one meltblown diehead is arranged near a chute through which other materials are added to the web while it is forming. Such other materials may be pulp, superabsorbent particles, natural polymers (for example, rayon or cotton fibers or other cellulosic materials) and/or

synthetic polymers (for example, polypropylene or polyester) fibers, for example, where the fibers may be of staple length. Coform processes are shown in commonly assigned US Patents 4,818,464 to Lau and 4,100,324 to Anderson et al. Webs produced by the coform process are generally referred to as coform materials.

A bonded carded web is made from staple fibers which are sent through a combing or carding unit, which breaks apart and aligns the staple fibers in the machine direction to form a generally machine direction-oriented fibrous nonwoven web. Once the web is formed, it then is bonded by one or more of several methods such as powder bonding, pattern bonding, through air bonding and ultrasonic bonding.

In the airlaying process, bundles of small fibers having typical lengths ranging from about 3 to about 52 millimeters (mm) are separated and entrained in an air supply and then deposited onto a forming screen, usually with the assistance of a vacuum supply. The randomly deposited fibers then are bonded to one another. Examples of airlaid teachings include the DanWeb process as described in US patent 4,640,810 to Laursen et al. and assigned to Scan Web of North America Inc, the Kroyer process as described in US patent 4,494,278 to Kroyer et al. and US patent 5,527,171 to Soerensen assigned to Niro Separation a/s, the method of US patent 4,375,448 to Appel et al assigned to Kimberly-Clark Corporation, or other similar methods.

The microbe contamination detection system may be incorporated on any variety of elastomeric articles. For example, gloves and other medical devices, such as dilatation balloons, inflatable cuffs, external catheters, catheter balloons, instrument covers, and so forth, may be utilized. The elastomeric article typically contains an elastomeric material formed from an elastomeric material, such as natural rubber latex, isoprene polymers, chloroprene polymers, vinyl chloride polymers, S-EB-S (styrene-ethylene-butylene-styrene) block copolymers, S-I-S (styrene-isoprene-styrene) block copolymers, S-B-S (styrene-butadiene-styrene) block copolymers, S-I (styrene-isoprene) block copolymers, S-B (styrene-butadiene) block copolymers, butadiene polymers, styrene-butadiene polymers, carboxylated styrene-butadiene polymers, acrylonitrile-butadiene polymers, carboxylated acrylonitrile-butadiene polymers, acrylonitrile-styrene-butadiene polymers, carboxylated acrylonitrile-styrene-butadiene polymers, derivatives thereof, and so forth. Suitable S-EB-S block copolymers, for instance, are described in U.S. Patent Nos. 5,112,900 to Buddenhagen, et al.; 5,407,715 to Buddenhagen, et al.; 5,900,452 to Plamthottam; and 6,288,159 to Plamthottam, which are incorporated herein in their entirety by reference thereto for all purposes.

The elastomeric article may be single- or multi-layered. For example, the article may include a coating that overlies at least a portion of the elastomeric material. In one particular embodiment, a donning layer may be used to facilitate the insertion of an elastomeric glove over a user's hand. Some examples of suitable materials for the donning layer include, but are not limited to, polybutadienes (e.g., syndiotactic 1,2 polybutadiene), polyurethanes, block copolymers, and so forth. Other examples of such polymers are described in U.S. Patent No. 5,792,531 to Littleton, et al., which is incorporated herein in its entirety by reference thereto for all purposes. A lubricant may also coat the donning layer to further aid in wet and/or dry lubricity. The lubricant, for example, may include a cationic surfactant (e.g., cetyl pyridinium chloride), an anionic surfactant (e.g., sodium lauryl sulfate), a nonionic surfactant (e.g., polyethylene glycol), and so forth. The lubricant may also contain a silicone emulsion, such as DC 365 (Dow Coming) or SM 2140 (GE Silicones).

An elastomeric article may generally be formed using a variety of processes known in the art. For example, elastomeric article formation techniques may utilize dipping, spraying, chlorination, drying, curing, as well as any other technique known in the art. Some examples of suitable methods for forming an elastomeric article that may be used are described in U.S. Patent Nos. 5,112,900 to Buddenhagen, et al.; 5,407,715 to Buddenhagen, et al.; 5,742,943 to Chen; 5,792,531 to Littleton et al.; 5,900,452 to Plamthottam; 6,288,159 to Plamthottam; and 6,306,514 to Weikel, et al., which are incorporated herein in their entirety by reference thereto for all purposes.

For instance, in some embodiments, a former having the shape of the article, such as a hand shape for an elastomeric glove, is initially dipped into a bath containing a coagulant for an elastomeric material. The bath may also include other optional ingredients, such as a surfactant, water, a salt that contains calcium ions (e.g., calcium nitrate and/or calcium carbonate), and so forth. The salt, for instance, may break the protection system of natural rubber latex emulsions and also facilitate removal of the tacky latex from the former, thus acting as a release agent. The surfactant may provide good wetting to avoid forming a meniscus and trapping air between the form and deposited latex, particularly in the cuff area. If desired, the former may be preheated so that the residual heat dries off the water leaving, for example, calcium nitrate, calcium carbonate, and surfactant on the surface of the former. Other suitable coagulant solutions are also described in U.S. Patent No. 4,310,928 to Joung, which is incorporated herein in its entirety by reference thereto for all purposes.

After being immersed in the coagulant composition, the former is withdrawn and allowed to dry. The former may then be dipped into a tank containing an elastomeric polymer bath to form the substrate body. The bath contains, for example, natural rubber latex, stabilizers, antioxidants, curing activators, organic accelerators, vulcanizers, and so forth. The former is dipped into one or more latex baths a sufficient number of times to build up the desired thickness on the former. By way of example, the substrate body may have a thickness of from about 0.1 to about 0.3 millimeters. If a glove is being formed, a bead roll station may be utilized to impart a cuff thereto. The latex-coated former is then dipped into a leaching tank in which hot water is circulated to remove the water-soluble components, such as residual

calcium nitrates and proteins contained in the natural latex. This leaching process may continue for about twelve minutes with the tank water being about 49°C. The article may then be optionally dipped into a solution to form a coating. Once coated, the former is sent to a curing station (e.g., oven) where the latex is vulcanized or cured. The elastomeric article may then be applied with various other treatment compositions (e.g., lubricant, halogenation, etc.), either "off-line" (i.e., after stripping) or "on-line".

Generally speaking, the microbe-sensitive chromogen possesses the ability to signal the presence of one or more microbes. Any of a variety of different types of microbes may generally be detected, such as bacteria, fungi, viruses, mold, yeast, etc. For example, the microbe-sensitive chromogens may detect bacteria of a variety of different shapes, cell arrangements, and compositions. Most bacteria, for instance, have one of five basic cell shapes, i.e., (1) round or cocci, (2) rod or bacilli, (3) spiral or spirilli, (4) comma or vibrios, and (5) filaments. Likewise, examples of possible cell arrangements include diplococci (e.g., pair), streptococci (e.g., chain), and staphylococci (e.g., bunched). Diplococci, for example, are known to cause pneumonia. Streptococci are often associated with "strep throat." Staphylococci are familiar to many because of their role in "staph infections" and some types of food poisoning. Bacteria also vary somewhat in size, but generally average about 1/25,000 inch (2.54 cm) per bacteria. In some cases, the shape or cell arrangement may impact the sensitivity of a particular chromogen for the microbe.

Although bacteria generally contain cell membranes (i.e., walls) made from lipid bi-layers ofliposaccharides, the composition of a type of bacteria may be more specifically classified using a Gram reaction (a staining method to classify bacteria). For example, gram-positive bacteria retain crystal violet stain in the presence of alcohol or acetone and include, for instance, the genera *Actinomyces, Bacillus, Bifidobacterium, Cellulomonas, Clostridium, Corynebacteriumk, Micrococcus, Mycobacterium, Nocardia, Staphylococcus, Streptococcus* and *Streptomyces.* Some of the Gram-positive bacteria notably those of the genera *Corynebacterium, Mycobacterium* and *Nocardia* retain dyes even in the presence of acid. These are known as Acid-Fast bacteria. Gram-negative bacteria do not retain crystal violet stain in the presence of alcohol or acetone, and include, for instance, the genera *Acetobacter, Agrobacterium, Alcaligenes, Bordetella, Brucella, Campylobacter, Caulobacter, Enterobacter, Erwinia, Escherichia, Helicobacterium, Legionella, Nesseria, Nitrobact, Pasteurelia, Pseudomonas, Rhizobium, Rickettsia, Salmonella, Shigella, Thiobacilus, Veiellonealla, Vibrio, Xanthomonas* and *Yersinia.*

Gram-negative cell membranes include lipopolysaccharides as a main component, and additionally include phospholipids, proteins, lipoproteins, and small amounts of peptidoglycans. The lipopolysaccharide component has a core region to which are attached repeating units of polysaccharide moieties or side chains. The chemical composition of these side chains, both with respect to composition and arrangement of the different sugars, determines the nature of the somatic or O antigen determinants. Such determinants, in turn, are useful in serologically classifying many gram-negative species. For example, some types of gram-negative bacteria that belong to quite different species and have strong serological cross-reactivity, nevertheless possess chemically similar carbohydrate moieties as part of their lipopolysaccharide side chains, which generally have about 30 repeating units. The cell membranes of gram-positive bacteria include peptidoglycans, polysaccharides, and/or teichoic acids. The peptidoglycans (also called "murein") are heteropolymers of glycan strands and are cross-linked through short peptides. The bases of the murein are chains of alternating residues of *N*-acetylglucosamine and *N*-acetyl muramic acid, which are β-1,4-linked. These chains are cross-linked by short polypetide chains containing both L- and D-amino acids.

Despite sharing common features, the arrangement and composition of the surfaces of gram-positive and gram-negative bacteria nevertheless differ. For example, gram-negative bacteria have an outer membrane coated with lipopolysaccharide (LPS). The LPS lends a net-negative charge to the surface of gram-negative bacteria and contributes to its pathogenesis. Gram-positive bacteria, on the other hand, are coated with thick peptidoglycan (or murein) sheet-like layers. The sheets are formed from alternating *N*-acetylglucosamine and *N*-acetylmuramic acid molecules. Teichoic acids are also found in gram-positive bacteria and may be linked to the *N*-acetylmuramic acid. While gram-negative bacteria also have peptidoglycan, the layer on gram-positive bacteria is much thicker. The peptidoglycan layer of gram-negative bacteria is also located underneath the LPS layer, making it less accessible from the surface.

In addition to bacteria, other microbes of interest include molds and yeasts (e.g., *Candida albicans*), which belong to the Fungi kingdom. Zygomycota, for example, is a class of fungi that includes black bread mold and other molds that exhibit a symbiotic relationship with plants and animals. These molds are capable of fusing and forming tough "zygospores." Ascomycota is another class of fungi, which includes yeasts, powdery mildews, black and blue-green molds, and some species that cause diseases such as Dutch elm disease, apple scab, and ergot. The life cycle of these fungi combines both sexual and asexual reproduction, and the hyphae are subdivided into porous walls that allow for passage of the nuclei and cytoplasm. Deuteromycota is another class of fungi that includes a miscellaneous collection of fungi that do not fit easily into the aforementioned classes or the Basidiomycota class (which includes most mushrooms, pore fungi, and puffball fungi). Deuteromycetes include the species that create cheese and penicillin, but also includes disease-causing members such as those that lead to athlete's foot and ringworm.

Thus, methods described herein involve the detection of bacteria and other microorganisms and the use of the term "microbe" herein should be understood to include bacteria, fungi like yeasts and molds, and viruses.

Regardless of the type of microbe of interest, a microbe-sensitive chromogen may be selected that interacts in some manner with the cell membrane of the microbe and/or the environment in which the microbe is present. As a result of this interaction, the chromogen undergoes a change in color that is readily detectable (e.g., with an unaided eye). The term "color" relates to the presence or absence of certain wavelengths of light reflected or emitted from objects in a visual field. Light entering the eye, for example, is subjected to a spectral analysis by three types of retinal cone cells that are sensitive to specific regions of the visible spectrum. Stimuli from these cells are in turn processed by retinal neurons, optic nerve neurons and the visual cortex so that a sensation of color is experienced. The chromogen typically owes its color to the absorption of certain wavelengths of light. As such, the perceived color is usually the complement of the color associated with the wavelength of light being absorbed by the object. An object that appears to be red in color when viewed in white light, for example, is in fact selectively absorbing bluish light in the wavelength range of 490 to 500 nanometers. Similarly, an object that appears yellow in white light is in fact absorbing blue light in the wavelength range of 435 to 480 nanometers.

In addition to visual detection of the color changes associated with the microbe-sensitive chromogen, the color changes may also be detected by an optical reader. The actual configuration and structure of the optical reader may generally vary as is readily understood by those skilled in the art. Typically, the optical reader contains an illumination source that is capable of emitting electromagnetic radiation and a detector that is capable of registering a signal (e.g., transmitted or reflected light). Changes in the registered signal indicate a change in color. The illumination source may be any device known in the art that is capable of providing electromagnetic radiation, such as light in the visible or near-visible range (e.g., infrared or ultraviolet light). The detector may generally be any device known in the art that is capable of sensing a signal. For instance, the detector may be an electronic imaging detector that is configured for spatial discrimination. Some examples of such electronic imaging sensors include high speed, linear charge-coupled devices (CCD), charge-injection devices (CID), complementary-metal-oxide-semiconductor (CMOS) devices, and so forth. The detector may be a light sensor that lacks spatial discrimination capabilities. For instance, examples of such light sensors may include photomultiplier devices, photodiodes, such as avalanche photodiodes or silicon photodiodes, and so forth. Optical readers may generally employ any known detection technique, including, for instance, luminescence (e.g., fluorescence, phosphorescence, etc.), absorbance (e.g., fluorescent or non-fluorescent), diffraction, etc.

The absorption of visible light is associated with electronic transitions within a molecule and results in the generation of an excited state. The energy difference between the ground state of the molecule and the relevant excited state determines the wavelength of the light absorbed according to the Planck relationship:

$$E=h\nu$$

wherein,

E is energy,

h is Planck's constant, and

$\nu$ is the frequency of the photon of light absorbed, and is related to wavelength $\lambda$ and the speed of light c by $\nu=c/\lambda$.

A state diagram, such as shown below, may be used to graphically depict electronic transitions:

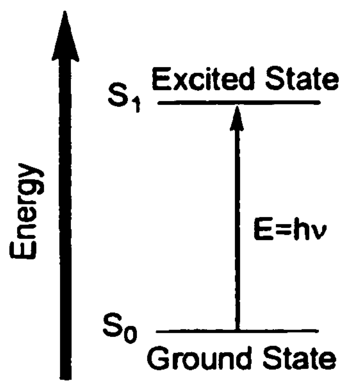


The energy of an absorbed photon is thus inversely proportional to the wavelength of the photon. Thus, photons of blue light (435 - 480 nanometers) have a higher energy than yellow light (580 - 595 nanometers). As such, the color of the chromogen is determined by the transition energy between the ground state of the chromogen and the first allowed excited state.

The light-absorbing portion of the chromogen employed in the microbe contamination detection system is a chromophore that is generally responsible for the color of the chromogen and is connected to a conjugated system. For instance, azo groups (e.g., azo dyes), polyene groups (e.g., carotene dye), carbonyl groups (e.g., anthraquinone dyes) are common chromophores. Auxochromes may induce a shift of the absorption maxima of the chromophore towards either the red end of the spectrum ("bathochromic shift") or the blue end of the spectrum ("hypsochromic shift"). Auxochromes may or may not be conjugated with the chromogen. For instance, an amino group conjugated to an azo group (chromophore) via, for instance, a benzene ring, will form an aminoazo chromogen. The type of absorption shift depends on the nature of the chromophore and, for example, on whether the auxochrome functions as an electron acceptor, in which a hypsochromic shift results, or whether the amino group functions as an electron donor, in which a bathochromic shift results. For example, a conjugated amino auxochrome will shift the absorption band of the azo group to longer wavelengths and increases the intensity of the absorption band. The absorption shift provides a color difference that is detectable, either visually or through instrumentation.

One particularly suitable class of chromogens that may undergo a detectable color change in the presence of one or more microbes is solvatochromic dyes. Solvatochromic dyes are dyes having spectroscopic characteristics (e.g., absorption) in the ultraviolet/visible/near-infrared spectrum and are sometimes influenced by the surrounding medium. The solvatochromic dyes may be positive or negative, which corresponds to bathochromic and hypsochromic shifts, respectively, of the emission band with increasing solvent polarity. In one embodiment, for instance, the solvatochromic dye undergoes a color change in a certain molecular environment based on solvent polarity and/or hydrogen bonding propensity. For example, a solvatochromic dye may be blue in color in a polar environment (e.g., water), but yellow or red in a non-polar environment (e.g., lipid-rich solution). The color produced by the solvatochromic dye depends on the molecular polarity difference between the ground and excited state of the dye.

Merocyanine dyes (e.g., mono-, di-, and tri-merocyanines) are one example of a type of solvatochromic dye that may be employed in the microbe contamination detection system. Merocyanine dyes, such as merocyanine 540, fall within the donor - simple acceptor chromogen classification of Griffiths as discussed in "Colour and Constitution of Organic Molecules" Academic Press, London (1976). More specifically, merocyanine dyes have a basic nucleus and acidic nucleus separated by a conjugated chain having an even number of methine carbons. Such dyes possess a carbonyl group that acts as an electron acceptor moiety. The electron acceptor is conjugated to an electron donating group, such as a hydroxyl or amino group. The merocyanine dyes may be cyclic or acyclic (e.g., vinylalogous amides of cyclic merocyanine dyes). For example, cyclic merocyanine dyes generally have the following structure:

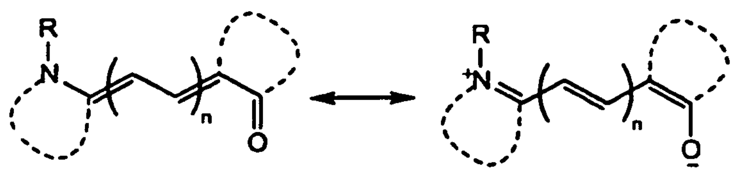

1 1'

wherein, n is any integer, including 0. As indicated above by the general structures 1 and 1', merocyanine dyes typically have a charge separated (i.e., "zwitterionic") resonance form. Zwitterionic dyes are those that contain both positive and negative charges and are net neutral, but highly charged. Without intending to be limited by theory, it is believed that the zwitterionic form contributes significantly to the ground state of the dye. The color produced by such dyes thus depends on the molecular polarity difference between the ground and excited state of the dye. One particular example of a merocyanine dye that has a ground state more polar than the excited state is set forth below as structure **2.**

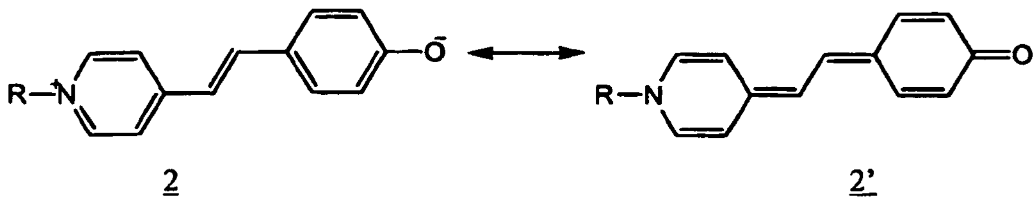

2 2'

The charge-separated left hand canonical **2** is a major contributor to the ground state whereas the right hand canonical **2'** is a major contributor to the first excited state. Still other examples of suitable merocyanine dyes are set forth below in the following structures **3-13.**

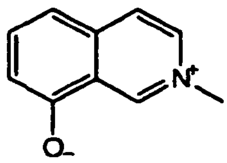

3

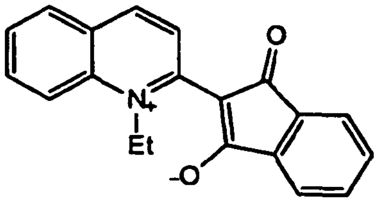

4

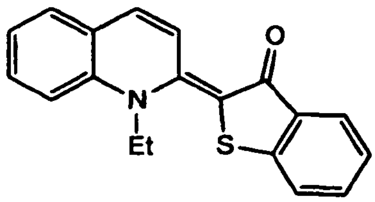

5

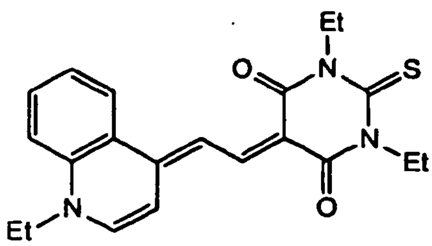

6

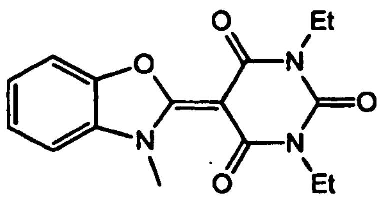

7

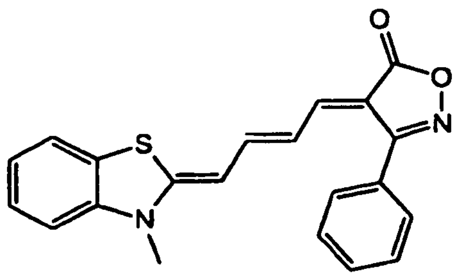

8

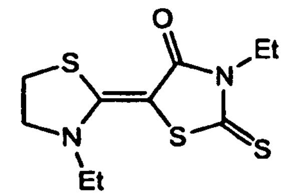

9

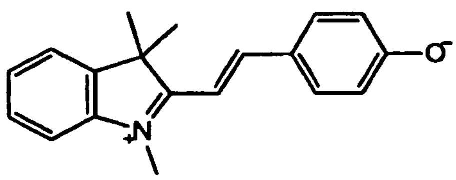

10

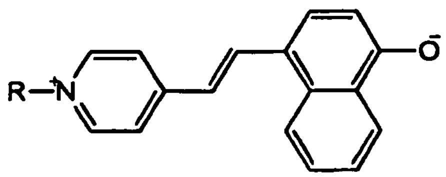

11

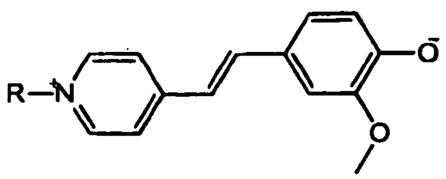

12

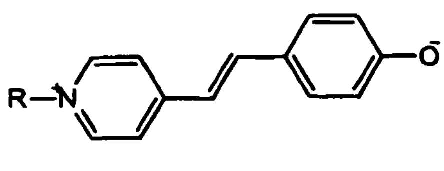

13

wherein, "R" is a group, such as methyl, alkyl, aryl, phenyl, etc.

Indigo is another example of a suitable solvatochromic dye. Indigo has a ground state that is significantly less polar than the excited state. For example, indigo generally has the following structure **14:**

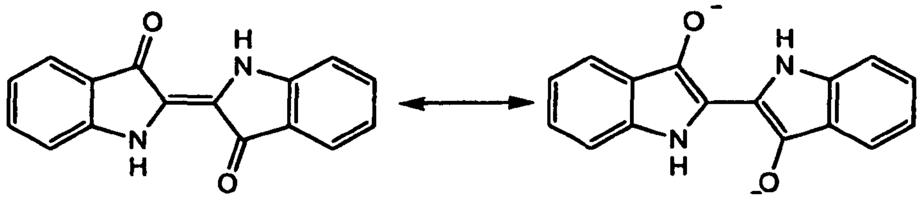

14 14'

The left hand canonical form **14** is a major contributor to the ground state of the dye, whereas the right hand canonical **14'** is a major contributor to the excited state.

Other suitable solvatochromic dyes include those that possess a permanent zwitterionic form. That is, these dyes have formal positive and negative charges contained within a contiguous π- electron system. Contrary to the merocyanine dyes referenced above, a neutral resonance structure cannot be drawn for such permanent zwitterionic chromogens. Exemplary dyes of this class include betaine dyes, such as 4-(2,4,6-triphenylpyridinium-1-yl)-2,6-diphenylphenolate (Reichardt's dye) having the following general structure **15.**

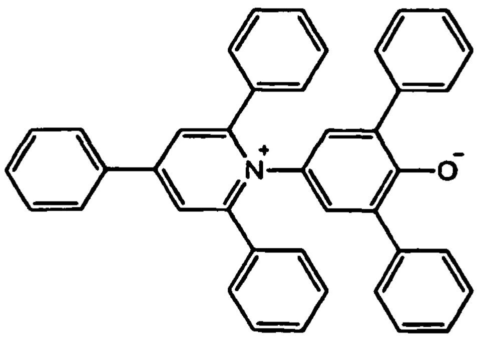

15

Reichardt's dye shows strong negative solvatochromism. That is, Reichardt's dye displays a shift in absorbance to a shorter wavelength and thus has visible color changes as solvent eluent strength (polarity) increases. Still other examples of suitable negatively solvatochromic pyridinium *N*-phenolate betaine dyes are set forth below in structures **16-22:**

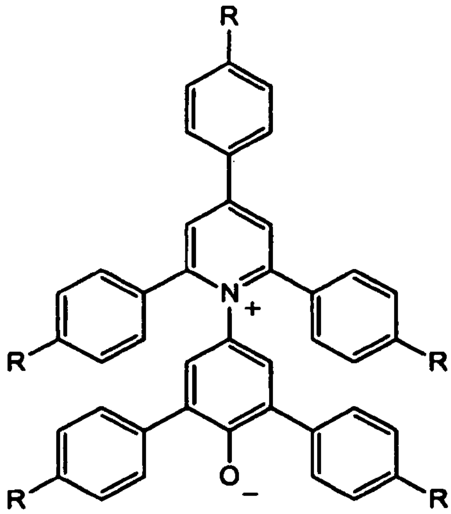

16

where R may be, for example, hydrogen, $-C(CH_3)_3$, $-CF_3$, or $C_6F_{13}$.

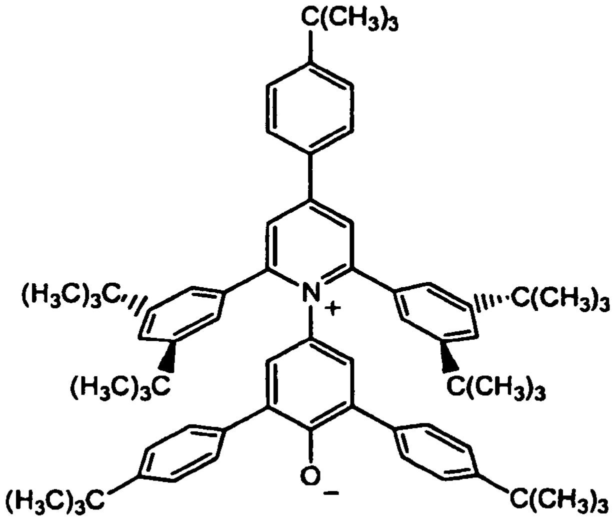

17

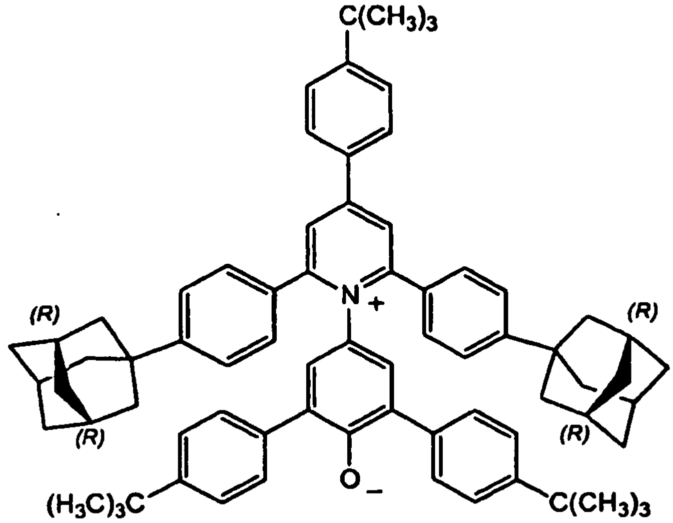

18

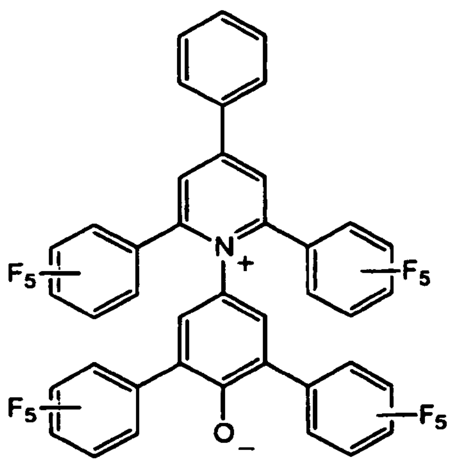

19

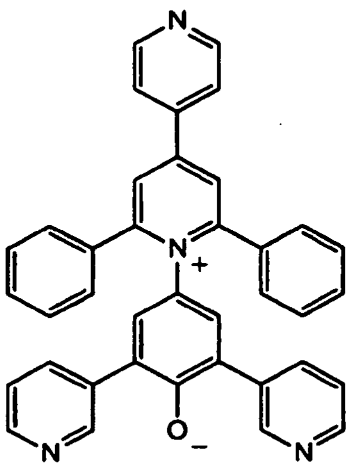

20

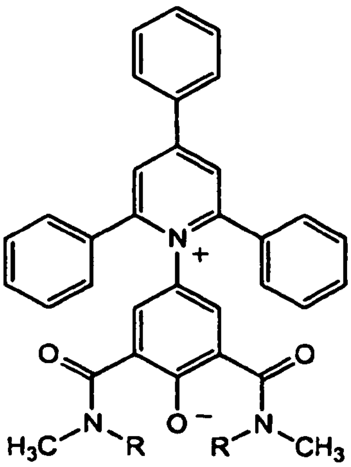

21

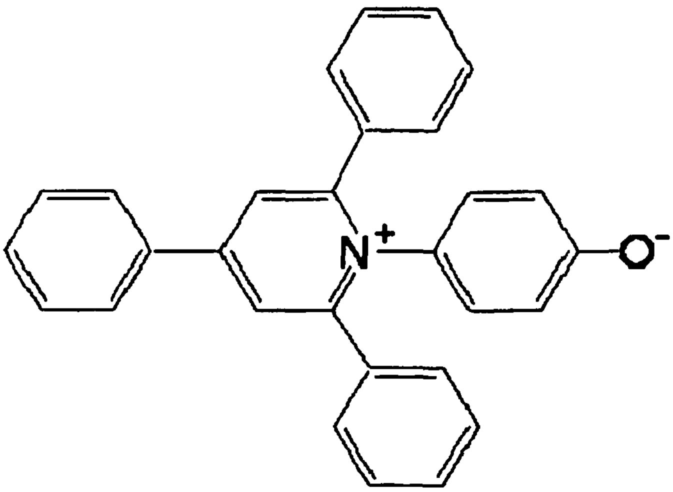

22

Still additional examples of dyes having a permanent zwitterionic form include dyes having the following general structure **23:**

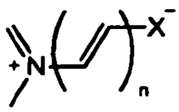

23

wherein, n is 0 or greater, and X is oxygen, carbon, nitrogen, sulfur, etc. Particular examples of the permanent zwitterionic dye shown in structure **23** include the following structures **24 - 32.**

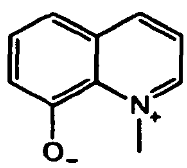

24

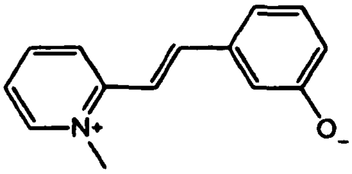

25

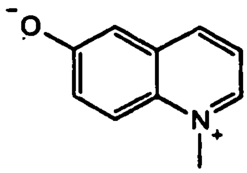

26

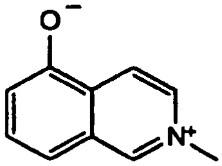

27

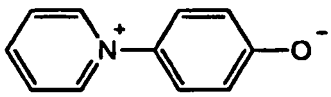

28

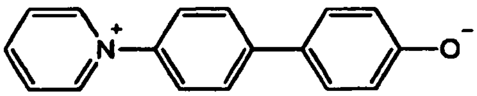

29

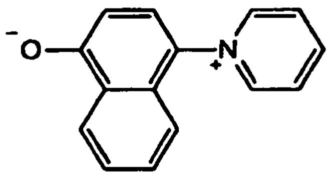

30

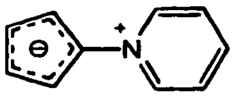

31

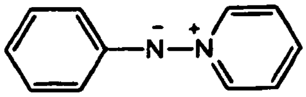

32

Other examples of solvatochromatic dyes may include, for instance: Reichardt's dye (available from Aldrich Chemical Co., Milwaukee, WI), 1-Docosyl-4-(4-hydroxystyryl)-pyridinium bromide (Aldrich Chem Co. Inc., Milwaukee WI), 2,6-dichloro-4-(2,4,6-triphenyl-N-pyridinio)-phenolate (hereinafter described as Betaine 1), 1-(4-hydroxyphenol)-2,4,6-triphenylpyridinium hydroxide (hereinafter described as Betaine 2), other pyridinium N-phenoxide betaines, and so forth. Reichardt's dye is a phenolbetaine, which shows very strong solvatochromism, the chemical structure of which is shown directly below ($R_1$-$R_5$ = phenyl):

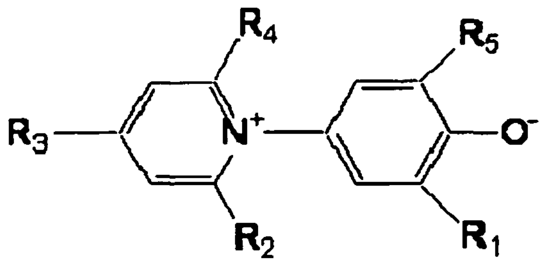

Even other microbe-sensitive dyes may be obtained by substitution of various groups for $R_1$ - $R_5$ in the betaine phenolate structure shown above. The $R_1$, $R_2$, $R_3$, $R_4$, and $R_5$ groups may be, for example, phenyl, benzyl, pyridinyl, aryl, heteroaryl, alkyl ($C_1$ - $C_{12}$), cycloalkyl, heterocyclo, halide, including chlorine, fluorine, and so forth, hydrogen, amido, amine, and thiol groups, and so forth.

Still other suitable solvatochromic dyes may include, but are not limited to 4-dicyanmethylene-2-methyl-6-(p-dimethylaminostyryl)-4H-pyran (DCM); 6-propionyl-2-(dimethylamino)naphthalene (PRODAN); 9-(diethylamino)-5H-benzo[a]phenox-azin-5-one (Nile Red); 4-(dicyanovinyl)julolidine (DCVJ); phenol blue; stilbazolium dyes; coumarin dyes; ketocyanine dyes; N,N-dimethyl-4-nitroaniline (NDMNA) and N-methyl-2-nitroaniline (NM2NA); Nile blue; 1-anilinonaphthalene-8-sulfonic acid (1,8-ANS), and dapoxylbutylsulfonamide (DBS) and other dapoxyl analogs. Besides the above-mentioned dyes, still other suitable dyes that may be used in the masking graphic include, but are not limited to, 4-[2-N-substituted-1,4-hydropyridin-4-ylidine)ethylidene]cyclohexa-2,5-dien-1-one, red pyrazolone dyes, azomethine dyes, indoaniline dyes, and mixtures thereof.

Although the above-referenced dyes are classified as solvatochromic, it should be understood that the present invention is not necessarily limited to any particular mechanism for the detectable color change of the chromogen in the presence of a microbe. For example, even when a solvatochromic dye is employed, other mechanisms may actually be wholly or partially responsible for the color change of the dye in the presence of a microbe. For example, acid-base or proton donation reactions between the dye and microbe may result in the color change. As an example, highly organized acid moieties on bacteria cell walls may protonate certain dyes, resulting in a loss of color. Redox reactions between the dye and microbe may likewise contribute to the color change.

The amount of microbe-sensitive dye must be sufficient to allow a change in color upon contact with microbes where the change is detectible to the unaided eye, and so will depend on the sensitivity of the dye. The amount of dye found to be sufficient is generally between 0.001 and 20 weight percent, in some embodiments between 0.01 and 10 weight percent, in some embodiments between 0.05 and 5, and in some embodiments, between 0.1 and 3 weight percent on a dry basis. The color change occurs quite rapidly in a manner that is dependent upon the concentration and type of microorganism.

Liquid or solvent containing the microbe-sensitive dye may be applied to surfaces and the applied liquid allowed to dry, forming the dried residue of the dye that may at a later time be exposed to contamination by microbes. Upon exposure to microbes, the dried residue will change color, indicating the presence of microbes. The color change may occur rapidly. For example, the period of time for the color change is desirably less than about five minutes, more desirably less than about one minute, even more desirably less than about ten seconds, and most desirably less than about one second. The time duration to turn colorless can also be controlled by varying the concentration of the microbe-sensitive dye used and the thickness of the resulting coating. Application of less dye will result in a shorter period of time to de-colorize. The period of time for de colorization is desirably short, thus minimizing the period of time from contamination to detection.

Bleaches used to clean surfaces like, for example, sodium hypochlorite solution, chlorine, and sodium bisulfite could possibly negatively impact solvatochromic dyes and cause a color change even though bacteria is not present. Another embodiment, therefore, includes a bleach detector colorant along with solvatochromic dyes. The bleach detector colorant could be, for example, 2,2',5,5'-tetramethyl benzidine, which is normally colorless and turns red when exposed to chlorine or sodium hypochlorite. The bleach detector colorant could also be a combination of starch and iodine which turns black in the presence of chlorine or hypochlorite. Yet another bleach detector colorant, fuchsine, may be useful for detection of sulfites, such as sodium metabisulfite. Fuchsine is pink and changes to colorless when exposed to sulfites. In this way, areas of the product may be designated as sensitive to bacteria and other areas as sensitive to bleaches and preservatives so that surfaces containing active bleach give color change combinations that allow the user to distinguish bacteria contamination from bleach. The bleach detector colorant could be printed in a pattern to spell the word "BLEACH", hidden on the product so that if the product were exposed to bleach, the word BLEACH would become visible, along with any other color change that the bleach may cause to the solvatochromic dye. The amount of bleach detector colorant need only be an amount sufficient to cause a color change that may be detected by the unaided eye and is in the same range as the solvatochromic dye.

In another aspect, a coating on the substrate may be used to inhibit the detecting dye(s) from crystallizing, thereby obtaining a coating that has greater sensitivity to microbes. Ideally, a coating that has single dye molecules on the surface would have greater sensitivity for microbes. Each dye molecule would be free to interact with the microbial membrane. In contrast, small crystals of dye first have to dissolve and then penetrate the membrane. While not wishing to be bound by theory, it is believed that hydroxypropyl-beta-cyclodextrin, hydroxyethyl-beta-cyclodextrin, gama-cyclodextrin, hydroxypropyl-gama-cyclodextrin, hydroxyethyl-gama-cyclodextrin (hereinafter collectively "cyclodextrin"), all available from Cerestar International of Hammond, IN, USA, hinder the crystallization of the dye, allowing a more vivid dye color to occur on the substrate. The amount of cyclodextrin has been found to be effective is between 0.001 and 2 weight percent, desirably between 0.01 and 1 weight percent and still more desirably between 0.025 and 0.5 weight percent.

Certain surfactants have also been found to assist dyes in detecting microbes. Particularly desired surfactants are nonionic surfactants, such as ethoxylated alkylphenols, ethoxylated and propoxylated fatty alcohols, ethylene oxide-propylene oxide block copolymers, ethoxylated esters of fatty ($C_8$ -$C_{18}$) acids, condensation products of ethylene oxide with long chain amines or amides, condensation products of ethylene oxide with alcohols, acetylenic diols, and mixtures thereof. Various specific examples of suitable nonionic surfactants include, but are not limited to, methyl gluceth-10, PEG-20 methyl glucose distearate, PEG-20 methyl glucose sesquistearate, $C_{11-15}$ pareth-20, ceteth-8, ceteth-12, dodoxynol-12, laureth-15, PEG-20 castor oil, polysorbate 20, steareth-20, polyoxyethylene-10 cetyl ether, polyoxyethylene-10 stearyl ether, polyoxyethylene-20 cetyl ether, polyoxyethylene-10 oleyl ether, polyoxyethylene-20 oleyl ether, an ethoxylated nonylphenol, ethoxylated octylphenol, ethoxylated dodecylphenol, or ethoxylated fatty ($C_6$ -$C_{22}$) alcohol, including 3 to 20 ethylene oxide moieties, polyoxyethylene-20 isohexadecyl ether, polyoxyethylene-23 glycerol laurate, polyoxy-ethylene-20 glyceryl stearate, PPG-10 methyl glucose ether, PPG-20 methyl glucose ether, polyoxyethylene-20 sorbitan monoesters, polyoxyethylene-80 castor oil, polyoxyethylene-15 tridecyl ether, polyoxy-ethylene-6 tridecyl ether, laureth-2, laureth-3, laureth-4, PEG-3 castor oil, PEG 600 dioleate, PEG 400 dioleate, and mixtures thereof. Commercially available nonionic surfactants may include the SURFYNOL® range of acetylenic diol surfactants available from Air Products and Chemicals of Allentown, Pennsylvania and the TWEEN® range of polyoxyethylene surfactants available from Fischer Scientific of Pittsburgh, Pennsylvania.

A binder may also be employed to facilitate the immobilization of the microbe-sensitive chromogen on a substrate. For example, water-soluble organic polymers may be employed as binders. One suitable class of water-soluble organic polymers includes polysaccharides and derivatives thereof. Polysaccharides are polymers containing repeated carbohydrate units, which may be cationic, anionic, nonionic, and/or amphoteric. In one particular embodiment, the polysaccharide is a nonionic, cationic, anionic, and/or amphoteric cellulosic ether. Suitable nonionic cellulosic ethers may include, but are not limited to, alkyl cellulose ethers, such as methyl cellulose and ethyl cellulose; hydroxyalkyl cellulose ethers, such as hydroxyethyl cellulose, hydroxypropyl cellulose, hydroxypropyl hydroxybutyl cellulose, hydroxyethyl hydroxypropyl cellulose, hydroxyethyl hydroxybutyl cellulose and hydroxyethyl hydroxypropyl hydroxybutyl cellulose; alkyl hydroxyalkyl cellulose ethers, such as methyl hydroxyethyl cellulose, methyl hydroxypropyl cellulose, ethyl hydroxyethyl cellulose, ethyl hydroxypropyl cellulose, methyl ethyl hydroxyethyl cellulose and methyl ethyl hydroxypropyl cellulose; and so forth.

After bacteria induced decolorization of certain microbe-sensitive chromogens, such as Reichardt's day, decolorization of these dyes may be reversed using a strong base such as, for example, sodium hydroxide.

As described above, the substrate may be formed from any of a variety materials capable of being treated with a dye. For example, the substrate may be formed from a film, paper, a nonwoven fabric, a knitted fabric, a woven fabric, foam, etc. In one particular embodiment, the substrate is a facestock material commonly employed in the manufacture of labels, such as paper, polyester, polyethylene, polypropylene, polybutylene, polyamides, etc. An adhesive, such as a pressure-sensitive adhesive, heat-activated adhesive, hot melt adhesive, etc., may be employed on one or more

surfaces of the facestock material to help adhere it to a desired article. Suitable examples of pressure-sensitive adhesives include, for instance, acrylic-based adhesives and elastomeric adhesives. In one embodiment, the pressure-sensitive adhesive is based on copolymers of acrylic acid esters (e.g., 2-ethyl hexyl acrylate) with polar co-monomers (e.g., acrylic acid). The adhesive may have a thickness in the range of from about 0.1 to about 2 mils (2.5 to 50 microns). A release liner may also be employed that contacts the adhesive prior to use. The release liner may contain any of a variety of materials known to those of skill in the art, such as a silicone-coated paper or film substrate. During use, the treated substrate and adhesive are peeled from the release liner. Thereafter, the adhesive is placed adjacent to a desired location to expose the treated substrate to the environment.

Generally, as depicted in the accompanying Figures 1a-c, a product 10 has an surface 12 for displaying a signal graphic 14. As shown in Fig. 1a, the signal graphic 14 is initially obscured by a masking graphic 16. As depicted in Fig. 1b, a microbial contaminant 18 that contaminates the product 10 will contact the masking graphic 16. As depicted in Fig. 1c, the microbial contaminant 18 reacts with the masking graphic 16, causing the masking graphic 16 to become at least partially transparent. The resulting transparency of the graphic 16 causes the signal graphic 14 to become visible.

The surface 12 may appear in any convenient shape, for example, in the general configuration of a strip, belt, or other linear conformation, in which one dimension is significantly greater or longer than another, or may substantially take the shape of the product upon which it resides. According to one design, the surface 12 may be incorporated as part of a protective garment. In another embodiment, the surface 12 may be in the form of a patch or other conformation having a wide and large surface area, which patch may be attached to a protective garment as a microbe contamination detection system.. As another option, the surface 12 may have a particular design, motif, or shape (e.g., circle, square, rectangle, triangle, polygon, sunburst, star, stripes, flower, animal, vegetable, or article (toy-shape or silhouette, hammer, wand, gun, sword, etc.)). When in the form of a patch, the surface 12 may be incorporated as part of the outer surface of a variety of products and articles, such as a garment, safety device, or absorbent article (e.g., diaper, inflatable float, pad). The surface 12 may be any substrate to which a signal graphic may be imparted and obscured by a microbe-sensitive dye. The substrate to which the graphic is applied may include, but is not limited to, paper, wood, a wood product or composite, woven fabrics, non-woven fabrics, textiles, films, plastics, and the like. In one aspect, the graphic may be applied to textile articles, such as cloth. The signal graphic may include any number of components known to those skilled in the art of creating graphics, including, but not limited to, pigments, inks, markers, dyes, stains, paints, colorants, and so forth. Desirably, the signal graphic will be insensitive to the microbe contamination that will cause a change in the masking graphic. In one embodiment, certain anthraquinone dyes that do not contain hydroxyl functionalities in the main ring structure of the dye, such as, for example, Acid Green 25, have been found to be fairly microbe-insensitive. In another embodiment, pigments, such as, for example, CCI Multigloss yellow base pigment have been found to be microbe-insensitive.

Broader applications of the microbe contamination detection system to applications such as, for example, health care garments and articles, diapers, and food service preparation protective garments are possible.

The microbe contamination detection system can be a disposable, self-contained tool, which can be used in virtually any environment where it is desired that a graphic be displayed after contact with a microbial contamination.

The following descriptions serve as illustrative examples of several fields in which the microbe contamination detection system may be employed. The microbe contamination detection system could be deployed as a stand-alone device or be part of a package assembly or kit with other articles or components to help in medical or clinical settings as a general microbe contamination detection system for notifying caregivers or patients when health care products have been exposed to a microbial contamination. The device may be an indicator in a glove, surgical or medical gowns, drapes, bandages or dressings. According to health care guidelines, health care workers should change gloves at least every 2 hours. However, this time may need to be substantially shorter in the event of microbial contamination of the gloves or other product. be seldom adhered to, leading to punctures and cross-contamination issues. This application is achieved by a simple masking graphic that fades upon exposure to microbial contamination to reveal a color, message, or graphic alerting the user to change the gloves.

The present invention may be better understood by reference to the Examples below. However, it is to be understood that the invention is not limited thereto.

Example 1

The use of chameleon type microbe-sensitive dyes to display signal graphics was demonstrated as follows. A CCI Multigloss yellow base pigment (available from Color Converting, Inc. of Hazleton, PA) was applied to a white SMS (spunbond/meltblown/spunbond) nonwoven material (available from Kimberly-Clark Corporation of Irving, TX) using a standard hand roller. The treated nonwoven was oven-dried at 100° C for approximately five minutes. A solution of Reichardt's dye was prepared by mixing about 200 mg of Reichardt's dye (obtained from Sigma-Aldrich of St. Louis, MO) with approximately 5 g of octanol (obtained from Sigma-Aldrich). This solution was then applied in the shape of a "t" to the dried yellow pigment on the nonwoven material using a clean hand roller. A control material was prepared by

applying the Reichardt's dye/octanol mixture in the "t" pattern to a sheet of untreated white SMS nonwoven material. The samples were again oven-dried at 100°C for approximately five minutes. In previous studies, it has been discovered that strong acids can also cause de-colorization of the dye in a manner that is representative of microbial contamination. Thus, strong acids are sometimes used to model the reaction of a microbe for initial experiments. A drop of pH 2 Aldrich buffer was applied to both the control and test substrates in three places and allowed to react for three minutes. After this time, the underlying yellow color was clearly visible. De-colorization of the control also occurred, revealing the white SMS underneath. Photos taken approximately 5 minutes after application of the buffer clearly showed the visible yellow color. An observation was made that the yellow color became even more visible as time passed (presumably due to the complete de-colorization of all dye molecules in the area of the drop as the acid dried).

Example 2

This experiment illustrated the ability of a surface coating of Reichardt's dye to respond to bacterial contamination. A sheet of paper was brush-coated with a Reichardt's dye solution (80 mg / 10 mL acetonitrile). To this paper was added 100 μL aliquots of $10^7$, $10^6$, $10^5$, and $10^4$ CFU/mL *E. coli* (ATCC #8739) or *S. aureus* (ATCC #6538) solutions. Water was used as a negative control. The dye color was rapidly discharged when contaminated by both types of bacteria, but more rapidly for the *S. aureus*. It was later determined that while both bacteria solutions were indeed at $10^7$ CFU/mL concentrations, the actual concentration of the *S. aureus* solution was 7 X $10^7$ CFU/mL compared to 1 X $10^7$ CFU/mL for the *E. coli* solution. Water caused slight decolorization of the dye after several minutes, in contrast to the rapid de-colorization (<1 min) observed for the bacteria solutions.

A sheet of paper self-adhesive stickers (Avery-Dennison) was also brush-coated with two different concentrations of Reichardt's dye solution (160 mg/10 mL acetonitrile, 80 mg /10 mL acetonitrile). The stickers were applied to the lid and latch mechanism of a Huggies® Wet Wipes box. A gloved hand was used to transfer $10^7$ CFU/mL *S. aureus* to the surface of the stickers. Though both concentrations were rapidly de-colorized, the color discharge was more easily visible on the surface which had the lower concentration of dye, indicating that there may be an optimal coating concentration that provides detection and strong visual contrast.

Example 3

This experiment illustrated the ability of a strong base to reverse decolorization of Reichardt's dye. Several drops of Reichardt's dye (160 mg in 10 ml of acetonitrile) were pipetted onto a SCOTT® towel and allowed to dry. Two compounds known to cause color changes (acetic acid and Aldrich buffer pH 2.0) were each dropped onto two of the spots which led to rapid de-colorization of the dye. A drop of 1 N NaOH was then pipette onto one of each of the spots, causing rapid re-colorization. The blue/purple color of Reichardt's dye returned after the 1 N NaOH was added.

Example 4

A solution of Reichardt's dye (80 mg/10 mL acetonitrile) and TWEEN® 80 (200 μL) polyoxyethylene surfactant (from Fischer Scientific, Pittsburgh, PA) was prepared. This solution was then used to coat a ceramic surface and allowed to air dry. A second solution of Reichardt's dye (80 mg /10 mL acetonitrile) without surfactant was placed on the surface and allowed to air dry as well. After drying, a drop of aged chicken juice known to have a high bacterial count was placed on each coating area. The area containing the TWEEN® 80 surfactant de-colorized at a much faster rate (>20-30 seconds) when compared to the area that did not contain the TWEEN® surfactant. Furthermore, the addition of TWEEN® surfactant allowed for easy removal of the dye from the surface. The addition of a small amount of water allowed for complete removal from the surface while the addition of water to the spot that did not contain the surfactant did not improve ease of removal from the surface.

Example 5

The behavior of Reichardt's dye coatings made using various different solvents was evaluated. Solutions of Reichardt's dye in acetonitrile, isopropanol, and xylenes were prepared and the solutions were used to coat SCOTT® kitchen rolls towels and allowed to air-dry. The treated towels had 100 μl aliquots of *S. aureus* placed on them and the coating was observed for a color change. Only the acetonitrile solution-based coating had a rapid de-colorization where the bacterial suspension was placed. The Reichardt's dye was observed to have an even color when dissolved in acetonitrile. No visible color change was observed with the other two solvent coatings. It was later found that the concentration of Reichardt's dye could be adjusted such that isopropanol could be utilized as a solvent for the dye. Though the color of the dye was less intense than that seen with acetonitrile, the decolorization in response to microbial contamination is readily and easily observed.

Example 6

A transparent film covering half of a fresh chicken on a polystyrene tray (from supermarket) was stored at room temperature for three weeks. The pale yellow juices that collected in the polystyrene tray were collected using a pipette and used for tests.

A 47-mg quantity of 1-docosyl-4-(4-hydroxystyryl)-pyridinium bromide (from Aldrich Chemical) was mixed with 10 g dimethylformamide. A small amount of solids remained after shaking and settling. The orange supernatant fluid was dropped onto woven cotton fabric of basis weight (29.2 cm x 20.3 cm = 6.888g) to make orange-yellow colored circles. One drop of 1N sodium hydroxide solution was added to one orange-yellow spot on the cotton fabric, changing the color from orange-yellow to a pinkish orange.

Aged chicken juice was spotted onto the orange-yellow spots on the cotton fabric, producing a color change to very pale yellow. The color change was rapid on cotton. Similarly, aged chicken juice was dropped onto the pinkish-orange areas on the cotton (dye + NaOH soln.) causing a similar color change from pinkish-orange to very pale yellow.

Example 7

N-methyl merocyanine dye was synthesized as follows. Methyl iodide was added slowly to a stirred solution of δ-picoline in 50ml of isopropanol in an ice -bath. After addition was complete, the reaction was heated to reflux and reflux continued for 2 hours. The solution was then chilled in an ice-bath and the precipitate filtered, and washed with chilled alcohol, on a Buchner funnel. The powder was then dried in the fume-hood for 2 hours. Yield of crude product was 18.6 grams. The crude product was not further purified and used directly for the next step.

N-methyl-δ-Picolone (9.4g, 0.04 mole), and vanillin (6.1 g, 0.04 mole) were all dissolved into 50 ml of ethanol with stirring. To this solution was added piperidine (3.4 g, 0.04 mole) and the mixture refluxed for 16 hours. The reaction mixture was then chilled in an ice-bath and the product filtered off using a Buchner funnel and washed with chilled ethanol.

The crude dye of structure **13** above, where R=methyl, was then stirred in 250 ml of 0.2 Molar potassium hydroxide solution for 60 minutes to form the zwitterion and then filtered off using a Buchner funnel. The dye was then crystallized from the minimum quantity of a 1:1 water/methanol mixture. The yield was 9.4g (98%).

Example 8

Female human urine was collected and stored in a glass jar at room temperature for 8 days. N-methyl merocyanine dye of the structure below was synthesized as described above. A 0.5-g quantity of the dye was dissolved in 20 ml deionized water and coated onto a SCOTT® kitchen roll paper towel by dipping the towel into the solution, allowing the excess to drip off, and then allowing the coated toweling to dry at ambient conditions. The paper towel was stained a deep orange color by the dye.

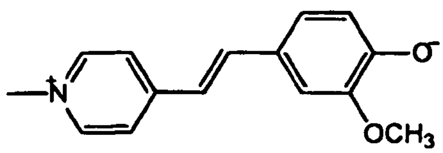

Aged urine was dropped onto the orange colored towel to give an immediate color change from deep orange to pale yellow. As a control, the aged urine was filtered through a 0.2 micron filter to remove bacteria and other microbes. After filtration, the aged urine did not cause a color change when dropped onto the towel suggesting that microbes were responsible for causing the color change vs. other components in the aged urine.

Example 9

Female human urine was collected and stored for 24 hours at 37°C. Pooled female urine may be expected to have a bacterial loading of approximately $1x10^5$ CFU / ml after storage under these conditions. A 0.5-g quantity of N-methyl merocyanine dye, synthesized as described above, was dissolved in 20 ml deionized water and coated onto a SCOTT® kitchen roll paper towel by dipping the towel into the solution, allowing the excess to drip off, and then allowing the coated toweling to dry at ambient conditions. The paper towel was stained a deep orange color by the dye.

The aged urine was dropped onto the orange colored towel to give an immediate color change from deep orange to pale yellow. As a control, the aged urine was filtered through a 0.2 micron filter to remove bacteria and other microbes. After filtration, the aged urine did not cause a color change when dropped onto the towel suggesting that microbes were responsible for causing the color change vs. other components in the aged urine.

Example 10

Budgerigar feces was collected from a caged pet budgie, and shaken in approximately 10 ml of Atlanta city domestic tap water. A 0.5-g quantity of N-methyl merocyanine dye, synthesized as described above, was dissolved in 20ml deionized water and coated onto a SCOTT® kitchen roll paper towel by dipping the towel into the solution, allowing the excess to drip off, and then allowing the coated toweling to dry at ambient conditions. The paper towel was stained a deep orange color by the dye.

Drops of the budgie feces suspension in tap water were spotted onto the coated towel and produced an immediate color change from deep orange to pale yellow where the suspension was added. As a control, Atlanta city domestic tap water was dropped onto a different area of the towel and while the color was diluted somewhat by the water, the area remained orange.

Example 11

A mixture of Reichardt's dye and 3,3',5,5'- tetramethylbenzidine (TMB) was coated onto a SCOTT® paper towel and allowed to air-dry. A dilute bleach solution was applied to the paper towel which resulted in the Reichardt's dye de-colorizing and the TMB turning orange/yellow color. This shows that a bleach indicator can be built into a microbe-sensitive graphic.

Additionally, a SCOTT® paper towel having a coating of Reichardt's dye and TMB chemistries was exposed to suspension of *E. coli* bacteria drop-wise. The towel area that came in contact with the bacteria decolorized to a white spot in less than 10 seconds. No orange/yellow color was observed to develop.

While the specification has been described in detail with respect to specific embodiments thereof, it will be appreciated that those skilled in the art, upon attaining an understanding of the foregoing, may readily conceive of alterations to, variations of, and equivalents to these embodiments. Accordingly, the scope of the present invention should be assessed as that of the appended claims and any equivalents thereto. Further, it is recognized that many embodiments may be conceived that do not achieve all of the advantages of some embodiments, yet the absence of a particular advantage shall not be construed to necessarily mean that such an embodiment is outside the scope of the present invention. In addition, it should be noted that any given range presented herein is intended to include any and all lesser included ranges. For example, a range of from 45-90 would also include 50-90; 45-80; 46-89 and the like. Thus, the range of 95% to 99.999% also includes, for example, the ranges of 96% to 99.1%, 96.3% to 99.7%, and 99.91% to 99.999%, etc.

**Claims**

1. A method of detecting the microbial contamination of a surface, the method comprising the steps of;
providing an article having a surface, the surface having thereon a signal graphic and a masking graphic overlying and obscuring the signal graphic, the masking graphic comprising a microbe-sensitive dye;
activating the microbe-sensitive dye by contact with a microbial contaminant to expose the signal graphic; and
detecting the exposed signal graphic.

2. The method according to claim 1, further comprising the step of taking an action in response to detection of the exposed signal graphic, such as wherein taking responsive action comprises one or more steps selected from the group consisting of cleaning the surface, disposing of the surface, and initiating an infection control protocol.

3. The method according to claim 1, wherein the signal graphic and the masking graphic cover less than the entirety of the surface.

4. The method according to claim 1, wherein a reaction between the microbe-sensitive dye and the microbial contaminant exposes the signal graphic or renders the masking graphic substantially transparent.

5. The method according to claim 1, wherein the signal graphic and the masking graphic are visually distinct from a background color of the surface.

6. The method according to claim 1, wherein the surface is on a major surface of a nonwoven material.

7. The method according to claim 6, wherein the nonwoven material is selected from the group consisting of film, spunbond, meltblown, SMS, coform, and airlaid materials.

**8.** The method according to claim 1, wherein the microbe-sensitive dye comprises a zwitterionic chromogen such as Reichardt's dye.

**9.** The method according to claim 1, wherein the microbe-sensitive dye is a merocyanine.

**10.** The method according to claim 1, wherein the microbe-sensitive dye comprises a compound having the formula

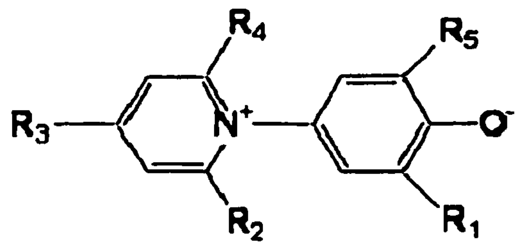

wherein R1, R2, R3, R4, and R5 are selected from the group consisting of phenyl, benzyl, pyridinyl, aryl, heteroaryl, alkyl (C1 - C12), cycloalkyl, heterocyclo, halide, chlorine, fluorine, hydrogen, amido, amine, and thiol groups.

**11.** The method according to claim 1, wherein the masking graphic and the signal graphic are differently colored.

**12.** A personal care article having a surface, the surface having thereon a signal graphic and a masking graphic overlying and obscuring the signal graphic, wherein the surface is a major surface of a nonwoven material, the nonwoven material being selected from the group consisting of spunbond, meltblown, SMS, coform and airlaid materials, and the masking graphic comprising a microbe-sensitive dye.

**13.** The personal care article according to claim 12, wherein the personal care article is a glove.

**Patentansprüche**

**1.** Verfahren zum Nachweisen der mikrobiellen Kontamination einer Oberfläche, wobei das Verfahren die Schritte umfasst:

Bereitstellen eines Artikels mit einer Oberfläche, wobei die Oberfläche darauf eine Signalgraphik und eine die Signalgraphik überlagernde und verbergende Maskierungsgraphik aufweist, wobei die Maskierungsgraphik einen Mikroben-empfindlichen Farbstoff umfasst;
Aktivieren des Mikroben-empfindlichen Farbstoffs durch Kontakt mit einer mikrobiellen Kontamination, um die Signalgraphik zu exponieren; und
Nachweisen der exponierten Signalgraphik.

**2.** Verfahren nach Anspruch 1, des Weiteren umfassend den Schritt in Reaktion auf den Nachweis der exponierten Signalgraphik eine Maßnahme zu ergreifen, wie etwa wobei das Ergreifen einer Reaktionsmaßnahme einen oder mehrere Schritte, ausgewählt aus der Gruppe, bestehend aus Reinigen der Oberfläche, Entsorgen der Oberfläche, und Einleiten eines Infektionsabwehrprotokolls, umfasst.

**3.** Verfahren nach Anspruch 1, wobei die Signal graphik und die Maskierungsgraphik weniger als die gesamte Oberfläche bedecken.

**4.** Verfahren nach Anspruch 1, wobei eine Reaktion zwischen dem Mikroben-empfindlichen Farbstoff und der mikrobiellen Kontamination die Signalgraphik exponiert oder die Maskierungsgraphik im Wesentlichen transparent macht.

**5.** Verfahren nach Anspruch 1, wobei die Signalgraphik und die Maskierungsgraphik von einer Hintergrundfarbe der Oberfläche visuell verschieden sind.

**6.** Verfahren nach Anspruch 1, wobei die Oberfläche auf einer Hauptoberfläche eines nicht-gewebten Materials ist.

7. Verfahren nach Anspruch 6, wobei das nicht-gewebte Material ausgewählt ist aus der Gruppe, bestehend aus Folienmaterial, Spunbond-Material, Meltblown-Material, SMS-Material, Coform-Material, und Airlaid-Material.

8. Verfahren nach Anspruch 1, wobei der Mikroben-empfindliche Farbstoff ein zwitterionisches Chromogen wie etwa Reichhardt-Farbstoff umfasst.

9. Verfahren nach Anspruch 1, wobei der Mikroben-empfindliche Farbstoff ein Merocyanin ist.

10. Verfahren nach Anspruch 1, wobei der Mikroben-empfindliche Farbstoff eine Verbindung mit der Formel

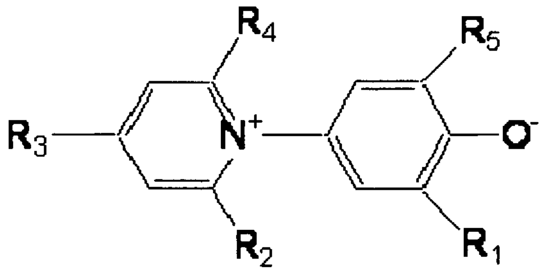

umfasst, worin R1, R2, R3, R4 und R5 ausgewählt sind aus der Gruppe, bestehend aus Phenyl-, Benzyl-, Pyridinyl-, Aryl-, Heteroaryl-, Alkyl- (C1-C12), Cycloalkyl-, Heterocyclo-, Halogen-, Chlor-, Fluor-, Wasserstoff , Amid-, Amin- und Thiolgruppen.

11. Verfahren nach Anspruch 1, wobei die Maskierungsgraphik und die Signalgraphik unterschiedlich gefärbt sind.

12. Körperpflegeartikel mit einer Oberfläche, wobei die Oberfläche darauf eine Signalgraphik und eine die Signalgraphik überlagernde und verbergende Maskierungsgraphik aufweist, die Oberfläche eine Hauptoberfläche eines nicht-gewebten Materials ist, das nicht-gewebte Material ausgewählt ist aus der Gruppe, bestehend aus Spunbond-Material, Meltblown-Material, SMS-Material, Coform-Material, und Airlaid-Material, und die Maskierungsgraphik einen Mikroben-empfindlichen Farbstoff umfasst.

13. Körperpflegeartikel nach Anspruch 12, wobei der Körperpflegeartikel ein Handschuh ist.

**Revendications**

1. Procédé de détection de la contamination microbienne d’une surface, le procédé comprenant les étapes de :

   fourniture d’un article ayant une surface, la surface portant sur elle un graphique de signal et un graphique de masquage se superposant au graphique de signal et l’obscurcissant, le graphique de masquage comprenant un colorant à sensibilité microbienne ;
   activation du colorant à sensibilité microbienne par contact avec un contaminant microbien pour exposer le graphique de signal ; et
   détection du graphique de signal exposé.

2. Procédé selon la revendication 1, comprenant en outre l’étape de prise d’une mesure en réponse à la détection du graphique de signal exposé, la prise de mesure appropriée comprenant une ou plusieurs étapes choisie(s) dans le groupe constitué par le nettoyage de la surface, la mise au rebut de la surface et le lancement d’un protocole de prévention de l’infection.

3. Procédé selon la revendication 1, dans lequel le graphique de signal et le graphique de masquage couvrent moins de la totalité de la surface.

4. Procédé selon la revendication 1, dans lequel une réaction entre le colorant à sensibilité microbienne et le contaminant microbien expose le graphique de signal ou rend le graphique de masquage sensiblement transparent.

**5.** Procédé selon la revendication 1, dans lequel le graphique de signal et le graphique de masquage sont visuellement distincts d'une couleur de fond de la surface.

**6.** Procédé selon la revendication 1, dans lequel la surface se trouve sur une surface principale d'une matière non tissée.

**7.** Procédé selon la revendication 6, dans lequel la matière non tissée est choisie dans le groupe constitué par les matières de film, les matières obtenues par filage-liage, par extrusion-soufflage, par SMS, par coformage et par voie aérodynamique (airlaid).

**8.** Procédé selon la revendication 1, dans lequel le colorant à sensibilité microbienne comprend un chromogène zwitterionique tel qu'un colorant de Reichardt.

**9.** Procédé selon la revendication 1, dans lequel le colorant à sensibilité microbienne est une mérocyanine.

**10.** Procédé selon la revendication 1, dans lequel le colorant à sensibilité microbienne comprend un composé ayant la formule

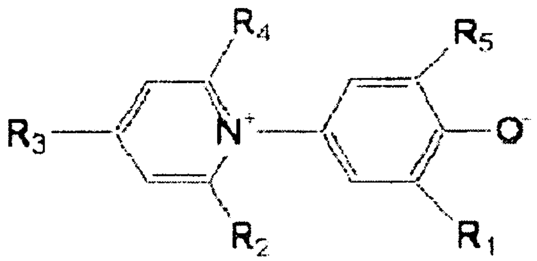

dans laquelle R1, R2, R3, R4, et R5 sont choisis dans le groupe constitué par les groupes phényle, benzyle, pyridinyle, aryle, hétéroaryle, alkyle (C1 - C12), cycloalkyle, hétérocyclo, halogénure, chlore, fluor, hydrogène, amido, amine et thiol.

**11.** Procédé selon la revendication 1, dans lequel le graphique de masquage et le graphique de signal sont colorés différemment.

**12.** Article pour soins d'hygiène personnelle ayant une surface, la surface portant sur elle un graphique de signal et un graphique de masquage se superposant au graphique de signal et l'obscurcissant, dans lequel la surface est une surface principale d'une matière non tissée, la matière non tissée étant choisie dans le groupe constitué par les matières obtenues par filage-liage, par extrusion-soufflage, par SMS, par coformage et par voie aérodynamique (airlaid), et le graphique de masquage comprenant un colorant à sensibilité microbienne.

**13.** Article pour soins d'hygiène personnelle selon la revendication 12, dans lequel l'article pour soins d'hygiène personnelle est un gant.

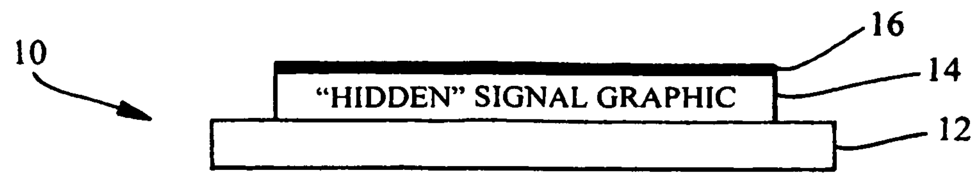


Fig. 1a

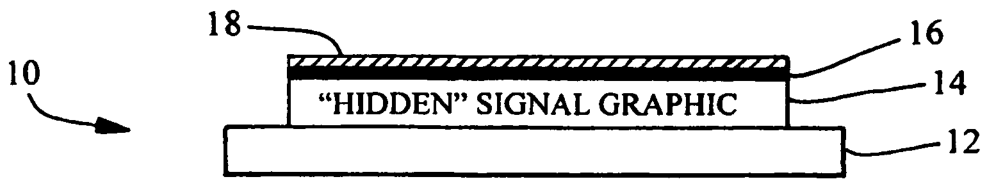


Fig. 1b

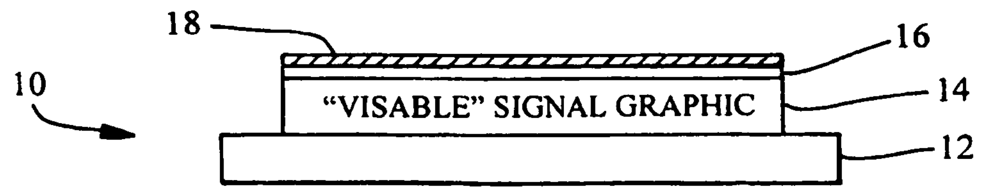


Fig. 1c

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006065349 A
- WO 2007027899 A
- EP 1919421 A
- US 4775582 A
- US 4853281 A
- US 4833003 A
- US 4511488 A
- US 4340563 A, Appel
- US 3692618 A, Dorschner
- US 3802817 A, Matsuki
- US 3338992 A
- US 3341394 A, Kinney
- US 3502763 A, Hartman
- US 3542615 A, Dobo
- US 3849241 A, Butin
- US 4818464 A, Lau
- US 4100324 A, Anderson
- US 4640810 A, Laursen
- US 4494278 A, Kroyer
- US 5527171 A, Soerensen
- US 4375448 A, Appel
- US 5112900 A, Buddenhagen
- US 5407715 A, Buddenhagen
- US 5900452 A, Plamthottam
- US 6288159 A, Plamthottam
- US 5792531 A, Littleton
- US 5742943 A, Chen
- US 6306514 A, Weikel
- US 4310928 A, Joung


### Non-patent literature cited in the description


- Colour and Constitution of Organic Molecules. Academic Press, 1976